# EUROPÄISCHE PATENTSCHRIFT

(11) **EP 0 283 882 B1**
(45) Veröffentlichungstag und Bekanntmachung des Hinweises auf die Patenterteilung: **19.01.2000**
(21) Anmeldenummer: 88103949.9
(22) Anmeldetag: 12.03.1988
(51) Int. Cl.: C12N 15/30, A61K 39/002, C07K 14/445, A61K 39/395

(54) **Klonierung von Malaria-spezifischen DNA-Sequenzen:Isolierung des Gens für das 140 kd Protein**
Cloning of malaria-specific DNA sequences: isolation of the 140 Kd protein gene
Clonage de séquences d'ADN spécifiques pour le paludisme: isolation du gène de la protéine 140 Kd

(30) Priorität: 18.03.1987 DE 3708783; 04.12.1987 DE 3741057
(43) Veröffentlichungstag der Anmeldung: 28.09.1988
(73) Patentinhaber: Chiron Behring Gmbh & Co., 35006 Marburg (DE)
(72) Erfinder: Knapp, Bernhard, Dr., D-3550 Marburg-Schröck (DE); Hundt, Erika, Dr., D-3550 Marburg-Wehrshausen (DE); Enders, Burghard, Dr., D-3550 Marburg (DE); Küpper, Hans, Dr., D-3550 Marburg (DE)
(74) Vertreter: Hallybone, Huw George

(56) Entgegenhaltungen:
- WO-A-84/02917
- WO-A-85/03725
- WO-A-86/04922
- BULLETIN OF THE WORLD HEALTH ORGANIZATION, Band 61, Nr. 1, 1983, Seiten 105-112; L. RODRIGUEZ DA SILVA et al.: "Plasmodium falciparum polypeptides released during in vitro cultivation"
- EXPERIENTIA, Band 42, Nr. 1, Januar 1986, Seiten 87,99, Birkenhäuser Verlag, Basel, CH; M. McGARVEY et al.: "Expression and production in E.coli of merozoite stage-specific polypeptide antigens from Plasmodium falciparum"
- THE EMBO JOURNAL, Band 6, Nr. 2, Februar 1987, Seiten 493-499, Irl. Press Ltd., Eynsham, Oxford, GB; F. ARDESHIR et al.: "A 75 kd merozoite surface protein of Plasmodium falciparum which is related to the 70 kd heat-shock proteins"
- PROCEEDINGS OF THE NATIONAL ACADEMY OF SCIENCES OF USA, Band 84, Nr. 5, März 1987, Seiten 1399-1403, Washington, DC, US; L. ASLUND et al.: "Synthetic gene construct expressing a repeated and highly immunogenic epitope of the Plasmodium falciparum antigen Pf155"

## Beschreibung

Malaria ist die am weitesten verbreitete und gefährlichste parasitäre Tropenkrankheit. Etwa 1/3 der gesamten Weltbevölkerung ist einem Infektionsrisiko ausgesetzt, ein schweres Gesundheitsproblem in etwa 60 Ländern Afrikas, Asiens und Lateinamerikas. Jährlich werden 150 Millionen Menschen infiziert, wobei allein im tropischen Afrika jährlich etwa 1 Mill. Kinder an Malaria sterben. (WHO, TDR 7th Program Report (1985)). Malaria wird durch den einzelligen Parasiten Plasmodium hervorgerufen, wobei vier Arten den Menschen befallen können: P. falciparum, P. vivax, P. malariae und P. ovale. P. falciparum verursacht die Malaria tropica, die gefährlichste Form. Die unbehandelte akute Erkrankung führt oft in wenigen Tagen zum Tod, besonders bei Kindern. Die Erreger werden durch den Stich der weiblichen Anophelesmücke auf den Menschen übertragen.

Die in den letzten Jahren rapide zunehmende und sich verbreitende Resistenz, sowohl des Überträgers (Mosquitos) gegen Insektizide als auch des Malariaparasiten P. falciparum gegen altbewährte und neu entwickelte Chemotherapeutika und Prophylaktika, zwingt die Wissenschaftler, neue Methoden zur Bekämpfung der Malaria zu entwickeln (WHO (1979) TDR, IMMAL, Vac 79, 1, Geneva). Die am meisten favorisierte Strategie ist die Entwicklung einer Malariavaccine zur aktiven Immunisierung. Dabei werden der gentechnologischen Herstellung einer oder mehrerer Plasmodium-Oberflächenproteine, die als Antigene einer aktiven Immunisierung dienen, die größten Chancen eingeräumt. Es ist nicht möglich, aus Parasitenkulturen entsprechende Antigene in genügend großen Mengen für eine konventionelle Vaccineproduktion zu gewinnen. Außerdem kann eine Unverträglichkeit des Impfstoffes, bedingt durch Kontamination mit Wirtszellen (erythrozytäre Bestandteile), nicht ausgeschlossen werden.

Durch den Stich einer infizierten Anophelesmücke gelangen Sporozoiten in den Blutkreislauf, welche sich umgehend in den Parenchymzellen der Leber festsetzen, vermehren und zu vielkernigen Gebilden (Schizonten) heranreifen. Durch Platzen dieser parasitierten Leberzellen werden Tausende von einkernigen Parasiten (sog. Merozoiten) freigesetzt und gelangen in das Blut. Diese Merozoiten sind ausschließlich auf den Befall von Erythrozyten spezialisiert, die an spezifischen Rezeptoren erkannt werden (erythrozytärer Zyklus). In den befallenen Erythrozyten machen die Parasiten verschiedene Entwicklungsstadien durch (genannt Schizogonie). Nach wiederholter Kernteilung füllen die Merozoiten den befallenen Erythrozyten vollständig aus. Durch das Platzen reifer Schizonten werden 18 - 24 Merozoiten pro Erythrozyt frei, die erneut Erythrozyten befallen und weitere Blutzyklen mit agamer Vermehrung initiieren. Der synchrone Ablauf dieser Zyklen, die je nach Malariaspezies 48 - 72 Stunden dauern, ist für die charakteristischen klinischen Malariasymptome (z.B. unregelmäßig hohes Fieber und Kopfschmerzen) verantwortlich. Nach mehreren Generationen im Blut differenzieren sich einige Parasiten zu weiblichen und männlichen Gametocyten (Geschlechtsformen), die von dem Mosquito bei einer Blutmahlzeit aufgenommen werden, sich im Magen des Insekts durch geschlechtliche Vermehrung zu infektiösen Sporozoiten entwickeln und sich später im Speichel ansammeln, um durch den Stich erneut in den menschlichen Wirtsorganismus zu gelangen.

Impfstoffe zur aktiven Immunisierung können im Prinzip innerhalb dieses Kreislaufes gegen das Sporozoiten-, das Merozoiten-, und/oder das Gametocytenstadium gerichtet sein. Eine Anti-Sporozoiten-Vaccine müßte absolut sicher sein und eine totale Inaktivierung der eingedrungenen Sporozoiten garantieren. Wenn nur ein einziger Sporozoit der Immunabwehr entkommt und eine Leberzelle erreicht, könnte eine ungestörte Entwicklung zu Merozoiten stattfinden, die eine völlig andere Antigenstruktur besitzen und deshalb durch anti-Sporozoit-Antikörper nicht neutralisiert werden können. Eine Gametocyten-Vaccine würde die sexuelle Vermehrung der Parasiten in der Anophelesmücke verhindern, so daß diese nicht mehr als Vektor im Zyklus fungieren kann. Geimpfte Personen wären daher nicht gegen eine Malaria-Infektion geschützt. Eine Merozoitenvaccine kann zu einer Impfung großer Bevölkerungsteile in Malaria-endemischen Gebieten eingesetzt werden und wäre sowohl in prophylaktischer als auch in therapeutischer Hinsicht von größerer Bedeutung.

Merozoiten besitzen eine große Anzahl verschiedener Proteinmoleküle, die als Impfantigene in Frage kämen, sowohl intrazellulär als auch auf der Zelloberfläche. Eine Immunreaktion gegen solche aktiven Antigenstrukturen konnte in vitro mit Malaria-Immunseren von Menschen oder immunisierten Tieren (Affen) bzw. mit monoklonalen Antikörpern festgestellt werden. Bisher sind die Isolierung und Charakterisierung einer ganzen Reihe von Plasmodienproteinen des asexuellen Blutstadiums beschrieben worden (D. J. Kemp et al. (1986) Parasitology 91, 83 - 108).

Als geeignetes Antigen zur Entwicklung einer rekombinanten Malaria-Vaccine dient eines der Proteine; die bei in vivo-Versuchen mit Saimiri- oder Aotus-Affen nach der Injektion einen Schutz vor einer letalen intravenösen P. falcinarum-Infektion ergeben haben, nämlich ein gelelektrophoretisch gereinigtes singuläres Parasitenprotein des Molekulargewichts 140 kd (L. B. Perrin et al. (1984), Clin. exp. Immunol. 56, 67-72; L. H. Perrin et al. (1984), J. Exp. Med. 160, 441-451). Von dem 140 kd Antigen wird angenommen, daß es als cytoplasmatisch lösliches Protein gebildet wird und in die Membran der Merozoiten und Schizonten posttranslationell integriert wird (C. Braun-Breton et al. (1986), Mol. and Biochem. Parasitology 20, 33-43). Bei den von Perrin et al. beschriebenen Antigenen (neben dem 140 kd-Protein auch Proteine von 200 und 41 kd) handelt es sich um Oberflächenproteine des Parasiten. Solche Antigene und auch Proteine, die auf der durch die Infektion veränderten Erythrozytenmembran lokalisiert sind, sind potentiell für einen gentechnologisch hergestellten Impfstoff gegen das asexuelle Blutstadium geeignet. Die Effektivität einer solchen Vaccine kann in in vivo-Versuchen in Saimiri- und/oder Aotus-Affen getestet werden.

Es ist nun von größter Bedeutung, von dem 140 kd-Antigen, von dem eine Schutzwirkung des isolierten Proteins bereits bekannt ist, die codierende DNA zu isolieren, in geeigneten Wirtszellen zu exprimieren und die Expressionsprodukte, sowie Teilsequenzen davon, in Immunisierungsexperimenten zu testen. Antigene, die sich als schützend erweisen, eignen sich zur Entwicklung einer Malariavaccine.

Gemäß einem ersten Aspekt betrifft die Erfindung Fragmente des 140 kd Antigens von *Plasmodium falciparum-*Merozoiten und -Schizonten mit den in Tabelle 2 angegebenen Aminosäureteilsequenzen der Positionen 1882 bis 1917, 2314 bis 2403 und 2602 bis 2631, die hydrophile Oberflächenbereiche sind, die potentielle antigene Epitope darstellen.

Gemäß einem zweiten Aspekt bezieht sich die Erfindung auf eine isolierte DNA- oder RNA-Sequenz, kodierend für mindestens ein Fragment nach dem ersten Aspekt.

Gemäß einem dritten Aspekt bezieht sich die Erfindung weiterhin auf einen Vektor, enthaltend mindestens eine Nukleotidsequenz nach dem zweiten Aspekt.

Gemäß einem vierten Aspekt betrifft die Erfindung weiterhin eine Wirtszelle, enthaltend einen Vektor nach dem dritten Aspekt.

Gemäß einem fünften Aspekt bezieht sich die Erfindung weiterhin auf ein Diagnostikum, enthaltend mindestens eine Nukleotidsequenz nach dem zweiten Aspekt.

Gemäß einem sechsten Aspekt betrifft die Erfindung ein Verfahren zur gentechnischen Herstellung von mindestens einem Teil des 140 kd Proteins, der ein hydrophiler Oberflächenbereich ist, der einen potentiellen antigenen Epitop darstellt, dadurch gekennzeichnet, daß mindestens eine Nukleotidsequenz nach dem zweiten Aspekt exprimiert und das Expressionsprodukt isoliert wird.

Zum Auffinden von Klonen, die für das 140 kd-Antigen kodieren, wurde eine λgt11-Gen-Bank mit einem monospezifischen Antiserum, im folgenden auch α140 kd-Serum genannt, das gegen das gelelektrophoretisch gereinigte Protein gerichtet ist, nach bekannten Methoden analysiert (L.S. Ozaki (1986), J. Immun. Method. 89, 213 - 219; Promega Biotec (1986), Proto Blot Immunoscreening System, Technical Manual).

Aus diesem "Screening" resultierten 2 Klone, 140-1 und 140-2, die durch das α140 kd-Serum erkannt wurden. Die Inserte dieser zwei Klone können mit EcoRI ausgeschnitten werden und umfassen 180 bzw. 150 bp. Durch Expression der malaria-spezifischen Nukleinsäure-Sequenzen der Phagen 140-1 und 140-2 als β-Galactosidasefusionsproteine und "Western Blot"-Analyse mit dem α140 kd-Serum, durch "Western Blot"-Analyse von Schizontenproteinen mit Seren, die gegen Expressionsproteine der Klone 140-1 und 140-2 gewonnen wurden sowie durch "Southern Blot"-Analyse von genomischer P. falciparum-DNA wurde nachgewiesen, daß beide Klone für unterschiedliche Teile des 140 kd-Antigens codieren.

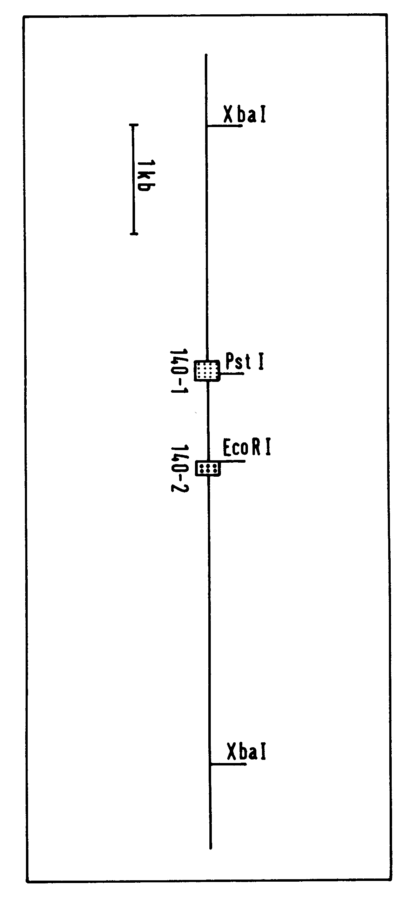

Durch DNA-Sequenzierung der Inserte 140-1 und 140-2 konnten sowohl DNA-Sequenzen des Gens, als auch Aminosäuresequenzen des 140 kd-Antigens bestimmt werden (Figur 1; die eingerahmten Sequenzabschnitte entsprechen den Linkern, die zur Herstellung der Genbank eingesetzt wurden). Beide Insertfragmente wurden mit Hilfe des P_{L}-Expressionsvektors pEX30b als MS2-Fusionsproteine zur Expression gebracht (K. Strebel et al., (1986) J. Virology 57, 983 - 991), welche im "Western Blot" mit dem monospezifischen α140 kd-Serum positiv reagierten.

Erfindungsgemäß können DNA-Abschnitte, die für das gesamte 140 kd-Antigen bzw. für Teilsequenzen codieren, dazu benutzt werden, Expressionsprodukte in geeigneten Wirtszellen herzustellen. Man könnte auch solche Expressionsprodukte in gereinigter Form zur Immunisierung von Tieren und Menschen einsetzen. Durch die Auswahl des Wirtes kann weiterhin die Form der Modifikation der Antigene beeinflußt werden. So findet in Bakterien keine, in Hefezellen eine andere Glykosylierung als in höheren eukaryontischen Zellen statt.

Weiterhin ist es aufgrund der bekannten Aminosäuresequenzen möglich, Oligopeptide herzustellen, die als Antigene zur Immunisierung dienen können.

Die Expressionsprodukte und synthetischen Oligopeptide können auch als Antigene zur Herstellung von polyklonalen oder monoklonalen Antikörpern dienen, welche in in vitro- oder in vivo-Inhibitionsassays zum Austesten von schützenden Epitopen Verwendung finden können. Solche neutralisierenden Antikörper können auch zur passiven Immunisierung gegen Malaria benutzt werden. Darüber hinaus können solche Antikörper zur Gewinnung des hochreinen 140 kd-Antigens sowie seiner gentechnologisch hergestellten Derivate eingesetzt werden. Mit Hilfe der beschriebenen DNA-Abschnitte oder mit Hilfe synthetisch hergestellter Oligonukleotidsonden kann man aus cDNA-Banken oder genomischen Banken weitere Klone isolieren, die weitere für die Antigene codierenden Teilstücke enthalten, welche sowohl zum 5'- als auch zum 3' -Ende dieser ermittelten DNA-Sequenzen hin expandieren. Deren Expressionsprodukte in verschiedenen Wirtssystemen können wiederum für Epitopuntersuchungen oder für Immunisierungsexperimente eingesetzt werden.

Weiterhin läßt sich das vollständige Gen für diese beiden Teilsequenzen isolieren, mit dessen Hilfe das gesamte 140 kd-Antigen oder zumindest größere Bereiche zur Expression gebracht werden können. Dazu können auch an solchen Klonen Mutationen in Form von Deletionen, z.B. von nicht codierenden Bereichen oder von nicht als schützend geeigneten codierenden Bereichen, sowie Insertionen und Basenaustausche vorgenommen werden.

Durch solche Manipulationen können relevante Proteinstrukturen zur Expression gebracht werden. Diese können dann in geeigneten in vitro- und in vivo-Testsystemen auf Schutzwirkung überprüft und einer klinischen Prüfungsphase zugänglich gemacht werden.

Man kann somit das 140 kd-Antigen sowie Teilsequenzen und mutierte Sequenzen beider Antigene gentechnologisch herstellen. Solche Expressionsprodukte oder synthetischen Peptide, welche als native Proteine Schutzwirkungen zeigen, können damit in genügend hoher Ausbeute erhalten werden und in reiner Form als Malaria-Vaccine gegen das asexuelle Blutstadium des Parasiten Verwendung finden.

Durch "Southern Blot"-Analysen von genomischer P.falciparum DNA des Stammes FCBR wurde nachgewiesen, daß die Insert-DNAs der Klone 140-1 und 140-2 werden auf einem genomischen XbaI-Fragment von 6,0 kb lokalisiert (Fig. 1) sind. Zur Isolation dieses genomischen DNA-Fragmentes wurde mit dem Restriktionsenzym XbaI geschnittene DNA im Größenbereich von 6,0 kb in die XbaI-Stelle des Phagenvektors λong C (Genofit GmbH, Heidelberg) kloniert. Durch Screening dieser Genbank mit den Insert-DNAs der Klone 140-1 und 140-2 resultierten mehrere positiv reagierende Klone. Die insertierte DNA eines dieser Phagenklone wurde in der XbaI-Stelle des Vektors pUC18 subkloniert und nach der Erstellung einer Restriktionskarte zu 2/3 sequenziert. Daraus konnte die gesamte Gensequenz und die abgeleitete Aminosäuresequenz des 140 kd Antigens aufgeklärt werden (Tab. 2).

In den folgenden Beispielen beziehen sich Prozentangaben auf das Gewicht, falls keine anderen Angaben gemacht sind.

### Beispiel 1:

### "Screening" einer λgt11-Expressions-Bank mit einem monospezifischen α140 kd-Serum

10⁶ PFU (plaque forming units) einer cDNA-λgt11-Expressionsbank (hergestellt aus mRNA des späten Schizontenstadiums des P. falciparum-Stammes SGE2) wurden einem "Screening" mit einem monospezifischen Antiserum gegen das 140 kd Antigen aus dem P. falciparum-Stamm SGE2 nach der Methode von R. A. Young und R. W. Davis ((1983), Proc. Natl. Acad. Sci. 80, 1194 - 1199) unterworfen. Dazu wurde eine Einzelkolonie des E. coli K12-Stammes Y 1090 in 25 ml LB-Medium, das 0,02 % Maltose enthielt, übertragen und bei 37°C für 5 h inkubiert. Die Zellen wurden abzentrifugiert und in SM (20 mM Tris-HCl, pH 7,5, 150 mM NaCl, 10 mM MgSO₄) resuspendiert, so daß die O.D.₆₀₀ 2,0 betrug. 10⁶ rekombinante Phagen der λgt11-Bank wurden mit 10 ml dieser Zellen gemischt und bei 37°C 20 min inkubiert. Je 200 µl dieser Phagen-Zellen-Suspension wurden mit je 4 ml Weich-Agar (0,7 % Agarose in LB) gemischt, auf 50 vortemperierte LB-Platten übertragen und 3 h bei 42°C inkubiert. Danach wurden diese LB-Platten auf 37°C gebracht und mit je einem trockenen auf 37°C vortemperierten numerierten Nitrocellulosefilter, der vorher in 10 mM IPTG (Isopropyl-β-D-thiogalactopyranosid) getaucht wurde, bedeckt. Nach einer weiteren 3-stündigen Inkubation bei 37°C wurden die Platten auf Raumtemperatur gebracht, die Position der Filter auf den Platten mit einer Nadel gekennzeichnet und die Filter nach vorsichtigem Abheben von den Platten 10 min bei Raumtemperatur in 250 ml Milchpuffer (5 % Milchpulver, 50 mM Tris-HCl, pH 8,0, 150 mM NaCl, 0,05 % nichtionisches Tensid (TWEEN 20)) sorgfältig geschüttelt. Die Filter wurden schließlich in frischen Milchpuffer transferiert und über Nacht bei 4°C aufbewahrt.

Nach weiterer zweimaliger Inkubation der Filter in je 250 ml Milchpuffer für je 10 min wurden die Filter mit 250 ml einer 1:250-Verdünnung in Milchpuffer des mit E. coli-Proteinen abgesättigten, in Kaninchen hergestellten α140 kd-Serums bei Raumtemperatur 1 h durch sorgfältiges Schütteln inkubiert. Die Filter wurden schließlich je viermal 10 min bei Raumtemperatur mit 250 ml Milchpuffer unter Schütteln gewaschen, bevor die Bindung mit dem anti-Kaninchen-IgG-alkalische Phosphatase-Konjugat (Promega, Bestell Nr. P 3731) durchgeführt wurde. Dazu wurden die Filter mit 250 ml einer 1:5000-Verdünnung in TBS (50 mM Tris-HCl, pH 8,0, 150 mM NaCl, 0,05 % TWEEN 20) eines anti-Kaninchen-IgG-alkalische Phosphatase-Konjugats der Konzentration 1 mg/ml 1 h bei Raumtemperatur inkubiert. Nach weiteren vier je zehnminütigen Waschschritten der Filter in je 250 ml TBS wurde die Farbreaktion nach der Methode des Herstellers (Promega) durchgeführt. Positiv blaue Signale auf den Filtern wurden den Regionen auf den Petrischalen zugeordnet, die jeweilige Region mit einer Pasteurpipette ausgestanzt und die Phagen in 1 ml SM resuspendiert. Positive Phagenplaques wurden über zwei weitere "Screening"-Schritte vereinzelt.

Da das α140 kd-Antiserum auch mit E. coli-Proteinen reagiert, mußte es zum "Screenen" der λgt11-Bank von E. coli-spezifischen Antikörpern gereinigt werden, um den "Background" zu reduzieren. Dazu werden λgt11-Phagen ohne Insertion zu einer Dichte von 5 x 10⁴ PFU pro 90 mm Agarplatte auf 30 LB-Agarplatten ausplattiert und über Nacht bei 37°C inkubiert. Auf jede Platte wurde ein Nitrocellulosefilter aufgelegt, die Platten 1 h bei 37°C inkubiert, die Filter herumgedreht und die Platten eine weitere Stunde bei 37°C inkubiert. Die Filter wurden schließlich in Milchpuffer zweimal 10 min bei Raumtemperatur inkubiert. 1 ml des α140 kd-Serums wurde danach mit 250 ml Milchpuffer verdünnt und dreimal je 1 h bei Raumtemperatur unter sorgfältigem Schütteln mit je 10 λgt11-Filtern inkubiert. Dieses 1:250 verdünnte und von E. coli-spezifischen Antikörpern befreite Serum konnte nach der Einstellung auf 0,01 % Natriumazid bei 4°C aufbewahrt werden. Diese Seren wurden sowohl zum "Screening" der λgt11-Bank als auch in den "Western Blots" verwendet.

Durch das "Screening" der cDNA-λgt11-Bank mit dem α140 kd-Serum konnten zwei intensiv reagierende λgt11-Klone, 140-1 und 140-2, identifiziert werden. Sie umfassen 180 bp bzw. 150 bp große Inserts, welche zur weiteren Charakterisierung in die EcoRI-Schnittstelle des Vektors pUC8 kloniert wurden. Zur eindeutigen immunologischen Zuordnung dieser Klone zu dem 140 kd-Antigen wurden die folgenden Experimente durchgeführt.

### Beispiel 2:

### Expression der β-Galactosidasefusionsproteine in lysogenen E. coli-Y1089-Zellen

Der E. coli K12-Stamm Y1089 (lac⁺, lon⁻, hflA 150) besitzt die Fähigkeit, die λgt11-rekombinante DNA in sein Genom stabil zu integrieren und durch Induktion der lacZ-Genregion mit IPTG als β-Galactosidasefusionsprotein zur Expression zu bringen. Deshalb wurden von den beiden Phagenklonen lysogene Zellen hergestellt und ihre β-Galactosidaseexpressionsprodukte analysiert (D.M. Glover (1985), DNA cloning I, a practical approach).

Von beiden Stämmen konnten β-Galactosidasefusionsproteine im SDS PAGE-Gel (U. K. Laemmli (1970), Nature 227, 680 - 685) beobachtet werden. Die elektrophoretisch aufgetrennten Proteine beider Stämme wurden auf Nitrocellulose transferiert (W. Neal Burnette (1981), Analytical Biochemistry 12, 195 - 203) und mit Hilfe von anti-β-Galactosidase-Antikörpern (anti-β-gal) und dem α140 kd-Serum untersucht. Dabei reagierten die Fusionsproteine und einige Abbauprodukte mit anti-β-gal. Mit Hilfe des α140 kd-Serums konnten die Fusionsproteine der Klone 140-1 und 140-2 durch eine schwache Reaktivität nachgewiesen werden.

### Beispiel 3:

### Expression der λgt11-Inserts in dem Vektor pEX30b

Mit Hilfe der Expressionsvektoren pEX30a, b, c (K. Strebel et al. (1986), J. Virology 57, 983 - 991) können DNA-Fragmente unter der Kontrolle des hitzeinduzierbaren P_{L}-Promotors, in den drei verschiedenen Leserahmen, nach der Methode von H. Küpper et al. ((1981) Nature 289, 555 - 559) als MS2-Polymerase-Fusionsproteine, zur Expression gebracht werden. Die EcoRI-Stelle des Vektors pEX30b besitzt dabei den gleichen Leserahmen wie die EcoRI-Insertionsstelle des Phagen λgt11. Daher wurden beide Inserts aus den pUC8-Vektoren durch das Restriktionsenzym EcoRI herausgeschnitten und nach gelelektrophoretischer Reinigung in die dephosphorylierte EcoRI-Stelle des Vektors pEX30b ligiert (T. Maniatis et al. (1982), Molecular Cloning, A Laboratory Manual). Die Ligationsansätze wurden in kompetente Zellen des modifizierten E. coli C600-Stammes 537 transformiert, welcher auf einem Plasmid das Kanamycin-Resistenzgen und das Gen für den temperatursensitiven Repressor cI857 trägt. Die Gewinnung kompetenter Zellen erfolgte mit Ausnahme der Kultivierung der E. coli 537-Zellen, die bei 28°C erfolgte, nach der CaCl₂-Methode. Die Transformation erfolgte 30 min in Eis, anschließend 2 min bei 42°C und nach Zugabe von 2 ml LB-Medium erfolgte die Regeneration der Zellen 1 h bei 28°C. Danach wurden die Zellen auf LB-Platten, die 50 µg/ml Ampicillin und 250 µg/ml Kanamycin enthielten, ausplattiert und über Nacht bei 28°C inkubiert. Pro Transformationsanzatz wurden 20 Einzelkolonien in je 5 ml auf 28°C erwärmtes LB-Medium mit 50 µg/ml Ampicillin und 25 µg/ml Kanamycin überführt und 20 Stunden bei 28°C heftig geschüttelt. Ein Aliquot jeder dieser Kulturen wurde 1:4 mit auf 42°C vorgewärmtes LB-Medium ohne Antibiotika verdünnt und 3 - 4 h bei 42°C heftig geschüttelt. Danach wurden 100 µl jeder Kultur vor und nach der Induktion pelletiert, das Pellet in 20 µl Puffer nach Laemmli (a. a. O.) resuspendiert, 5 min aufgekocht und auf 15 %igen SDS-Polyacrylamidgelen aufgetrennt. Im mit COOMASSIE®-Blau (ICI) gefärbten Gel wurden schließlich die Proteinfraktionen vor und nach der Induktion auf Expressionsprodukte untersucht. Es konnten beide Genfragmente als MS-2-Fusionsproteine in hoher Ausbeute zur Expression gebracht werden. Die Molekulargewichte der Expressionsprodukte betragen dabei 32 bzw. 22 kd. Der Expressionsklon, welcher das 140-1 DNA-Fragment integriert hat, enthält dieses DNA-Fragment dreifach. Sind diese drei Fragmente in der gleichen Orientierung integriert, so läßt sich das Molekulargewicht des Fusionsproteins von 32 kd erklären.

Durch Analyse der Expressionsprodukte mit dem monospezifischen 140 kd-Serum konnten die MS2-Fusionsproteine der Klone 140-1 und 140-2 mit Hilfe eines "Western Blots" deutlich nachgewiesen werden.

### Beispiel 4:

### "Western Blots" von Schizontenproteinen mit Antiseren gegen die Expressionsprodukte der Klone 140-1 und 140-2

Die MS2-Fusionsproteine der Klone 140-1 und 140-2 wurden aus dem Gesamtbakterienextrakt über Waschschritte mit ansteigenden Harnstoffkonzentrationen angereichert. Dazu wurde von beiden Expressionsklonen eine Kultur in 1 l LB-Medium mit 50 ng/ml Ampicillin und 25 µg/ml Kanamycin bei 28°C 20 Std. heftig geschüttelt. Nach Zugabe von 4 l auf 42°C erwärmtes LB-Medium wurde erneut 4 h bei 42°C geschüttelt. Die Bakterien wurden schließlich abzentrifugiert, in 200 ml phosphatgepufferter Kochsalzlösung (PBS) resuspendiert und mechanisch aufgeschlossen. Die löslichen Proteine wurden durch Zentrifugation abgetrennt und die Sedimente, die die Expressionsprodukte enthielten, nach zweimaligem Waschen mit PBS in 1 M Harnstoff resuspendiert. Die solubilisierten Proteine wurden durch Zentrifugation abgetrennt und die Sedimente erneut in 2 M Harnstoff suspendiert. Diese Schritte wurden mit steigenden Harnstoffkonzentrationen wiederholt. Bei einer Konzentration von 4 M Harnstoff gingen beide Fusionsproteine in Lösung und waren zu einer Reinheit von 70 - 80 % angereichert.

Kaninchen wurden nach dem folgenden Schema immunisiert: Tag 1: 1 mg in komplettem Freunds Adjuvant, subcutan; Tag 2 - 5, 15 - 19, 29 - 33, je 0,1 mg ®AEROSIL (Degussa) intravenös; Tag 40: Blutentnahme.

Zur Gewinnung von Schizonten wurde P. falciparum des Stammes FCBR in Humanerythrozyten kultiviert (Trager und Jensen (1976), Science 193, 673 - 675) und durch Behandlung mit Sorbit synchronisiert (Lambros und Vanderberg (1979), J. Parasitol. 65, 418 - 420). Eine Anreicherung der Schizonten auf ca. 90 % wurde durch Flotation in GELAFUNDIN® (Braun Melsungen) erzielt (analog Pasvol et al. (1978), Ann. Trop. Med. Paras. 72, 87 - 88). Die Schizonten wurden abzentrifugiert (5 min, 3000 g), in einer Pufferlösung unter Zusatz von Proteaseinhibitoren resuspendiert und in Aliquots bei -70°C gelagert.

Aliquots der Schizontensuspension wurden in SDS-Probenpuffer gelöst und mit Hilfe der SDS-PAGE aufgetrennt (Puffersystem nach Laemmli, a. a. O., Acrylamidgradient von 5 % bis 17,5 %). Die Schizontproteine wurden auf Nitrocellulose transferiert, die Membran in 1 cm breite Stücke geschnitten, welche nach zweimaligem Waschen mit Milchpuffer für je 10 min mit den isolierten Antiseren gegen die Expressionsproteine der Klone 140-1 und 140-2 und mit dem 140 kd-Antiserum für 1 h bei Raumtemperatur inkubiert wurden. Nach vier Waschschritten der Filter in Milchpuffer für je 10 min wurden die an spezifische Schizontproteine gebundenen Antikörper mit Hilfe eines Peroxidase-IgG-Konjugats lokalisiert.

Dabei reagiert das monospezifische α-140 kd-Serum hauptsächlich mit einer Proteinbande des Molekulargewichts 113 kd. Die Antiseren gegen die Expressionsproteine der Klone 140-1 und 140-2 erkennen ebenfalls das 113 kd-Protein. Vor allem das Antiserum gegen das Fusionsprotein des Klons 140-2 zeigt zusätzliche Kreuzreaktionen mit einer Reihe niedermolekularer P. falciparum-Proteine.

### Beispiel 5:

### Sequenzierung der Insert-Fragmente

Die Sequenzierung erfolgte sowohl nach der Methode von Maxam und Gilbert (Maxam und Gilbert (1980), Meth. Enzymol. 65, 499) als auch nach der Didesoxy-Methode mit Hilfe eines "Primer" und eines "Reverse Primer" direkt aus den pUC8-Plasmiden (E. Y. Chen, und P. H. Seeburg (1985), DNA 4, 165 - 170).

Die beiden cDNA-Fragmente der Klone 140-1 und 140-2 codieren für hydrophile Bereiche von 43 und 36 Aminosäuren des 140 kd-Antigens (Tab. 1). Die 23 bp umfassende identische Sequenz am 3ʹ-Ende der beiden Inserte und am 5ʹ-Ende von 140-1 entspricht der Linkersequenz, die für die Insertion der cDNA in den Vektor λgt10 benutzt wurde. Die Inserte der λgt10-Genbank wurden zur Herstellung einer Expressionsbank in die EcoRI-Schnittstelle des Vektors λgt11 kloniert. Auf mindestens eine solche Schnittstelle innerhalb des 140 kd-Gens lassen auch zwei unterschiedliche mit den Insert-DNAs der Klone 140-1 und 140-2 hybridisierende genomische EcoRI-Fragmente schließen.

### Beispiel 6:

### "Southern Blots" genomischer P. falciparum-DNA

Je 15 µg genomische DNA des Plasmodium falciparum-Stammes FCBR, die durch Lyse von Schizont-Kulturen mit anschließender Ethidiumbromid-CsCl-Zentrifugation gewonnen wurden (P. Oquendo et al. (1986), Molecular and Biochemical Parasitology 18, 89 - 101), wurden durch verschiedene Restriktionsenzyme verdaut, nach der Vorschrift des Herstellers auf "Gene Screen"-Membranen (DuPont) transferiert und anschließend mit "nicktranslatierter" (P. W. J. Rigby et al. (1977), J. Mol. Biol. 113, 237) Insert-DNA der spezifischen Aktivität von 10⁷ bis 10⁸ dpm/µg oder "Primer"-markierter (Analytical Biochemistry 137, 266 - 257 (1984)) Insert-DNA der spezifischen Aktivität von 10⁹ dpm/µg der Klone 140-1 und 140-2 hybridisiert. Nach dem Waschen der Filter in 0,3 x SSC (1 x SSC : 0,15 M NaCl, 0,015 M Natriumcitrat) und 1 % SDS bei 65°C für 1 h wurden die Filter autoradiographiert. Mit Hilfe der "Southern Blot"-Experimente wurde ein ca. 6 kb großes genomisches XbaI-Fragment identifiziert, das mit beiden Insert-DNAs der Klone 140-1 und 140-2 hybridisiert. Dies zeigt, daß beide Insert-Fragmente auf einem Gen lokalisiert sind und daß nur ein einziges Gen im Genom von P. falciparum existiert, das für das 140 kd-Protein kodiert.

Weitere "Southern-Blot"Experimente erlaubten die Lokalisierung der Insert-DNAs der Klone 140-1 und 140-2 auf dem genomischen XbaI-Fragment (Fig. 1). Dies wurde erleichtert duch das Vorhandensein einer PstI-Restriktionsschnittstelle innerhalb der Insert-DNA des Klons 140-1 und einer EcoRI-Schnittstelle innerhalb der Insert-DNA des Klons 140-2. Da die Insert-DNAs der Klone 140-1 und 140-2 etwa zur Mitte des genomischen XbaI-Fragmentes zugeordnet werden können und Intron-Sequenzen innerhalb der bisher bekannten charakterisierten Gene von P. falciparum sehr klein sind oder auch fehlen, kann davon ausgegangen werden, daß das genomische XbaI-Fragment das gesamte 140 kd-Gen oder zumindestens den größten Teil des Gens abdeckt. Diese Zuordnung der beiden Insert-Fragmente auf dem P. falciparum-Genom bietet nun die Voraussetzung zur Isolierung des 140 kd-Gens.

### Beispiel 7:

### Klonierung des 6,0 kb XbaI-Fragmentes

60 µg genomische DNA des P. falciparum-Stammes FCBR wurden mit dem Restriktionsenzym XbaI verdaut und gelelektrophoretisch aufgetrennt. DNA-Fragmente von 5,5 kb bis 6,5 kb wurden durch Elektroelution (B. Perbal (1984), a practical guide to molecular cloning) wiedergewonnen und nach dem Protokoll des Herstellers (Genofit) in die XbaI-Restriktionsstelle des Vektors λong C kloniert. Von der somit erhaltenen Phagen-Genbank wurden 10⁵ rekombinante Phagenklone nach bekannten Methoden (T. Maniatis et al. (1982), Molecular cloning, a laboratory manual) mit nicktranslatierter Insert-DNA des Klons 140-1 bzw. 140-2 analysiert. Daraus resultieren 20 Phagenklone, die mit beiden Insert-DNAs hybridisierten. Nach Gewinnung der Phagen-DNA eines dieser Klone wurde die 6,0 kb umfassende Insert-DNA in die XbaI-Stelle des Vektors pUC18 subkloniert (Klon pUC140 gen). Durch "Southern Blot"-Analyse des letzteren Klons mit nicktranslatierter Insert-DNA der Klone 140-1 und 140-2 wurde schließlich bestätigt, daß die Sequenz des Klons 140-1 diese pstI-Schnittstelle des 3,1 kb umfassenden XbaI-EcoRI-Restriktionsfragmentes beinhaltet und die Sequenz des Klons 140-2 auf dem 2,7 kb großen EcoRI-Restriktionsfragment lokalisiert ist (vgl. Fig. 1).

### Beispiel 8:

### Restriktionskartierung und Sequenzierung des 6,0 kb XbaI-Fragmentes

Mit Hilfe der Restriktionsenzyme XbaI und PstI und EcoRI, EcoRI und XbaI wurden aus dem Plasmid pUC140gen DNA-Fragmente der Länge 2,3 kb, 0,8 kb und 2,7 kb präpariert und in den Vektor pUC18 subkloniert. Je 0,3 µg der Insert-DNAs dieser Subklone wurden mit einer großen Auswahl von Restriktionsenzymen inkubiert, gelelektrophoretisch aufgetrennt und auf Nitrocellulosefilter geblottet. Diese wurden anschließend mit nicktranslatierter Insert-DNA der λgt11-Klone 140-1 und 140-2 hybridisiert, gewaschen und exponiert. Da die Sequenzen beider Phagenklone jeweils dem Randbereich der Insert-DNAs der präparierten Subklone bezüglich der bekannten PstI bzw. EcoRI-Stelle zugeordnet werden konnten, konnte aufgrund der Länge eines hybridisierenden Restriktionsfragmentes auf die Entfernung von der bekannten PstI bzw. EcoRI-Stelle zu der zu bestimmenden Restriktionsstelle geschlossen werden. Weiterhin wurden von dem 2,7 kb EcoRI-XbaI-Fragment mehrere größere Restriktionsfragmente isoliert und auf die Lokalisation von weiteren Restriktionsstellen untersucht. Die Erstellung einer solchen Restriktionskarte bot die Voraussetzung zur Subklonierung von Restriktionsfragmenten in die Phagen-DNAs M13mp18 und M13mp19, sowie in den Plasmidvektor pUC18 zum Zwecke der Sequenzierung nach der Didesoxykettenabbruch-Reaktionsmethode (F. Sanger et al. (1977), Proc. Natl. Acad. Sci. USA 74, 5463-5467; E. Y. Chen and P. H. Seeburg (1985) DNA 4, 165-170). Dabei wurden etwa 2/3 des 6,0 kb umfassenden XbaI-Fragments beidsträngig sequenziert und somit die gesamte für das 140 kd Antigen codierende Sequenz aufgeklärt.

Die komplette DNA-Sequenz und die abgeleitete Aminosäuresequenz des 140 kd Antigens ist in Tab. 2 dargestellt. Sowohl das nicht codierende 5ʹ-Ende als auch das nicht codierende 3ʹ-Ende dieses Gens (Position 1 bis 301 bzw. 3706 bis 3975) sind sehr AT-Reich (AT-Gehalt = 92,4 % bzw. 87,4 %), wie es für nicht codierende Regionen von P. falciparum Genen vorhergesagt wird (J. L. Weber (1987), Gene 52, 103-109). Die codierende Region vom ATC-Startcodon (Position 302) bis zum TAA-Stopcodon (Position 3706) hat einen AT-Gehalt von 60,6 % und wird durch 3 Intron-Sequenzen (Position 336 bis 487, Position 1280 bis 1454, Position 1596 bis 1720) in 4 Exon-Abschnitte unterteilt. Alle drei Introns verfügen über einen hohen AT-Gehalt von durchschnittlich 86,5 % und über mehrere Stopcodons in allen drei Leserahmen. Die Randsequenzen der drei Introns lassen sich weitgehend in die Consensus-Sequenz einfügen, welche für eine große Anzahl von Introns anderer Organismen bestimmt wurde (S. M. Mount (1982), Nucleic Acids Res. 10, 459-472). Die Positionen aller drei Introns wurden durch S1-Nuclease-Kartierung (A. J. Berk and P. A. Sharp (1977) Cell 12, 721-732) und durch RNA-Sequenzierung (BRL-Focus, 9, No. 1 (1987) 5-8) mit Hilfe von Nukleotidprimern, die etwa 30 Basen "downstream" der vorhergesagten Intron-Sequenzen an die mRNA binden, bestätigt.

Die codierende Region enthält 2952 Nucleotide; das entspricht 984 Aminosäuren oder einem Molekulargewicht von 110 kd. Dies ist in guter Übereinstimmung mit dem im "Western-Blot" ermittelten Molekulargewicht von 113 kd. Das erste Exon codiert dabei lediglich für 11 Aminosäuren. Mit den folgenden 5 Aminosäuren, die von dem zweiten Exonabschnitt codiert werden, bildet diese Sequenz eine typische hydrophobe Signal-Sequenz. Ähnliche Signal-Sequenzen wurden auch für andere Plasmodien Proteine beschrieben (A. A. Holder et al. (1985), Nature 317, 270-273; D. Simmons et al. (1987), The EMBO J. 6, 485-491; J. V. Ravetch et al. (1984), Nature 312, 616-620). Dieser Signal-Sequenz folgt unmittelbar ein repetitiver Sequenzabschnitt (Position 520 bis 663), der für sechs Octapeptide der Consensus Sequenz Ser X Ser Thr-Gly-Gly-Gly-Gln-Ala-Gly-Asn codiert. Dabei ist der Threonin-Rest in der 1. Position und der Glycin-Rest in der 7. Position extrem konserviert. Ein zweiter repetitiver Sequenzabschnitt des 140 kd Antigens von Position 1000 bis 1113 besteht aus 19 Hexanucleotiden der Sequenz-AGTTCTA- und codiert für eine repetitive Region, welche bis auf einen Aminosäure-Rest ausschließlich aus Serin-Resten besteht. Das dritte Exon, welches genauso wie das erste Exon sehr klein ist, codiert für nur 47 Aminosäuren und enthält keine repetitiven Sequenzabschnitte. Dies gilt auch für das größte, nämlich das vierte Exon. Dies beginnt in Position 1721 und beinhaltet die restliche für 662 Aminosäuren codierende Region des Gens. Dieses Exon enthält Sequenzabschnitte in Position 3339 bis 3469 und Position 2182 bis 2290, welche identisch sind mit den cDNA-Sequenzen der Klone 140-1 und 140-2 (siehe Tab. 1). Da die genomische Sequenz und die beiden cDNA-Sequenzen von unterschiedlichen P. falciparum-Stämmen herrühren, scheinen diese Abschnittte des 140 kd Antigens sehr konserviert zu sein.

Die Aminosäuresequenz des 140 kd Antigens wurde durch das UWGCG (University of Wisconsin Genetics Computer Group) Programm auf mögliche Glykosylierungsstellen, hydrophile- und Oberflächenbereiche, sowie antigen wirkende Regionen untersucht. Mögliche Glykosylierungsstellen sind dabei die Asparagin-Reste in den Aminosäurepositionen 176, 193, 305 und 815 des 984 Aminosäure umfassenden 140 kd Antigens. Hyrophile Oberflächenbereiche die potentielle antigene Epilope darstellen, findet man hauptsächlich in dem vom vierten Exon codierent Aminosäurebereichen der Positionen 1882 bis 1917, 2314 bis 2403 und 2602 bis 2631.

### Beispiel 9:

### "Southern Blot"-Analyse unterschiedlicher P. falciparum Stämme

DNA von vier geographisch verschiedenen Isolaten der P. falciparum Stämme FCBR (Kolumbien), Palo Alto (Uganda), ItG₂G₂ (Brasilien) und SGE-2 (Zaire) wurden mit den Restriktionsenzymen XbaI, EcoRI, XbaI und EcoRI, PvuII, PvuII und HgiAI, HincII und AccI, sowie NsiI verdaut, auf 0,8 %igen Agarosegelen aufgetrennt, auf Nitrocellulosefilter geblottet und mit ³²P markierter DNA von codierenden Bereichen des 140 kd Gens hybridisiert. Die verdaute genomische DNA aller vier Isolate zeigt dabei das gleiche Hybridisierungsmuster mit Banden identischer Größe. Diese Daten bestätigen, daß die Sequenz des 140 kd Gens bei all diesen Stämmen konserviert ist. Durch "Western-Blot" Analyse verschiedener P. falciparum Isolate (FCBR, Kolumbien; SGE2, Zaire, FVOR, Vietnam) mit dem 140 kd Antiserum konnte ebenfalls gezeigt werden, daß das 140 kd Antigen in seiner Größe und Sequenz konserviert bleibt.

Weiterhin zeigen die Ergebnisse, daß bei allen Isolaten nur eine einzige EcoRI-Restriktionsstelle (Position 2645 von Tab. 2) innerhalb des 6,0 kb XbaI-DNA-Fragmentes lokalisiert ist. Somit könnte die EcoRI-Stelle des Klons 104-2 (Tab. 1) dadurch erklärt werden, daß die ersten beiden Nukleotide (GA) dieses Inserts durch die benutzten DNA-Linker eingeführt wurden und somit zufällig eine EcoRI-Stelle entstand.

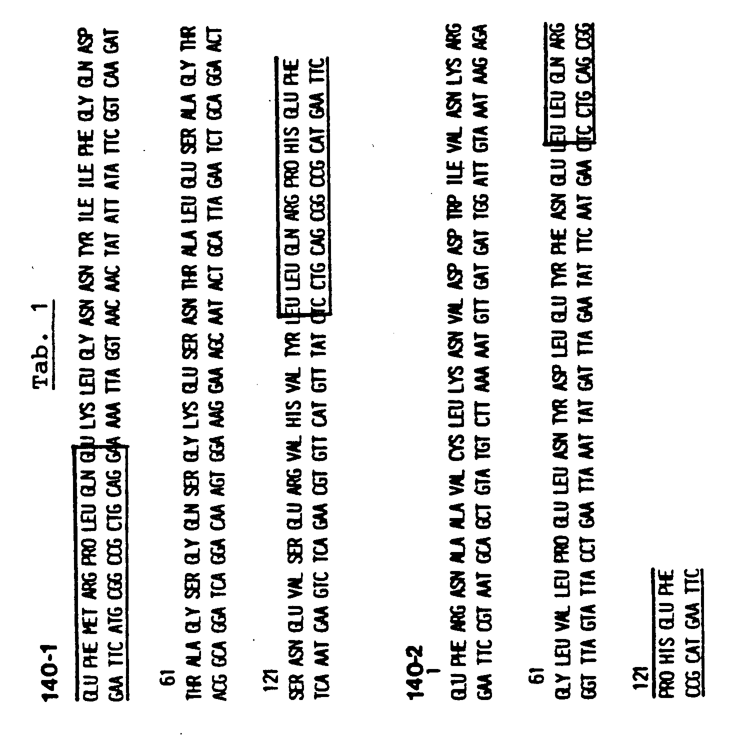

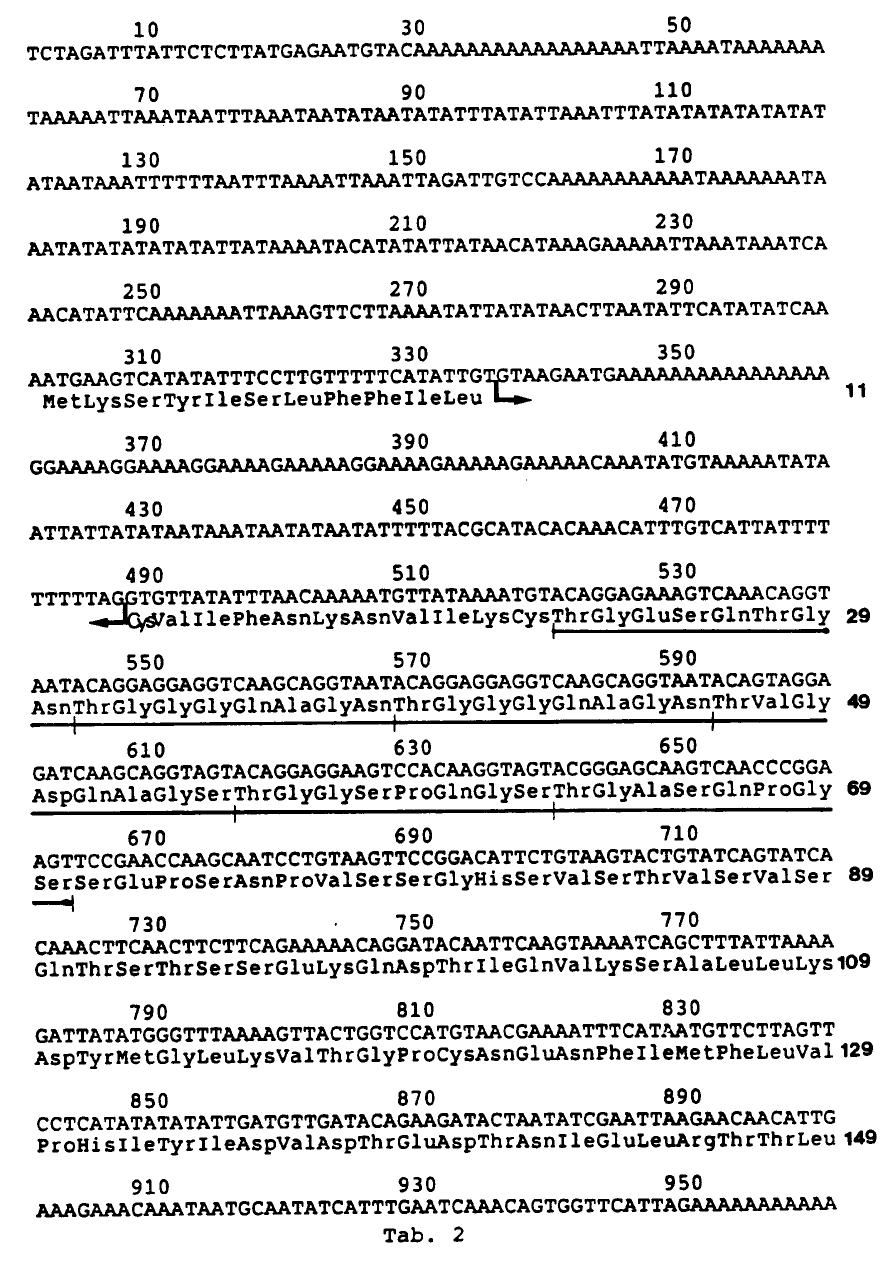

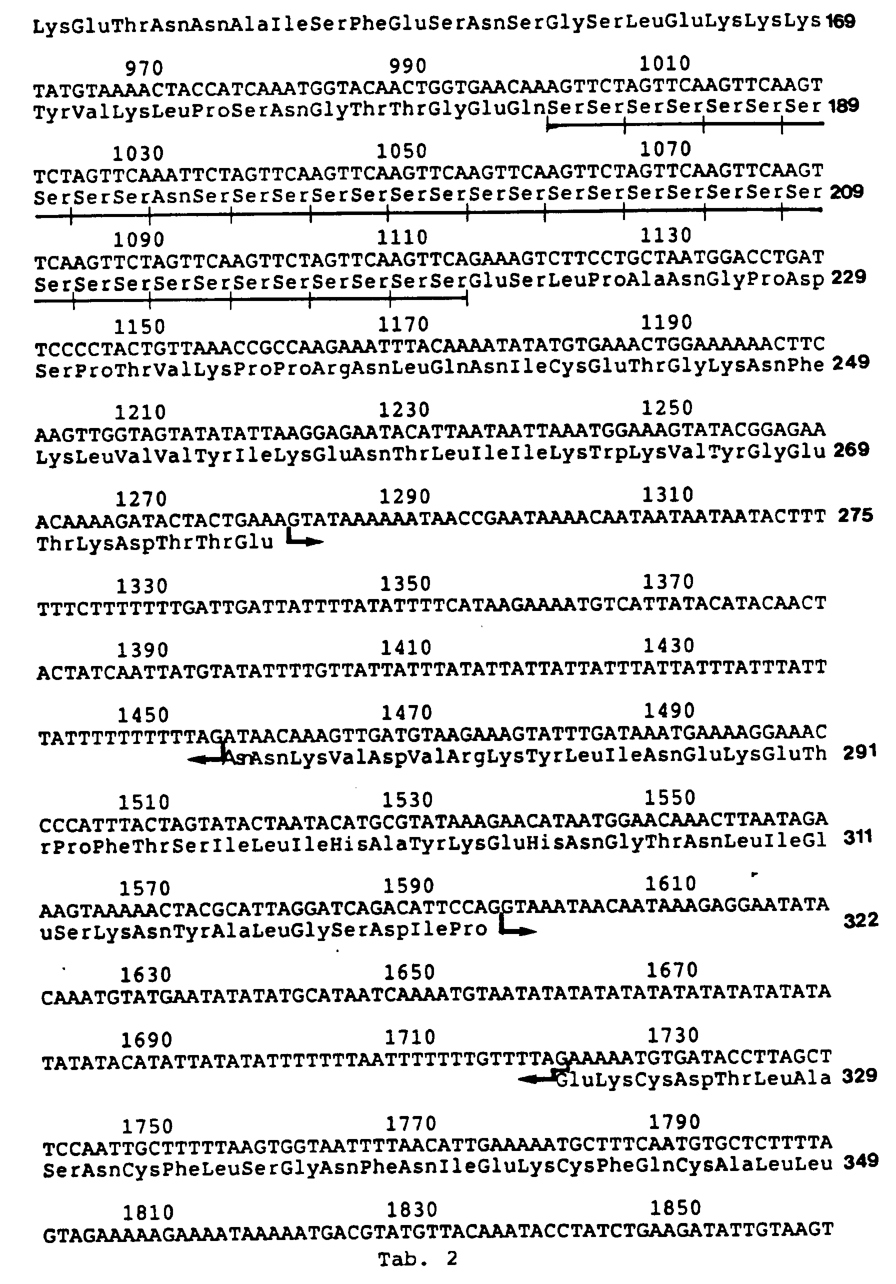

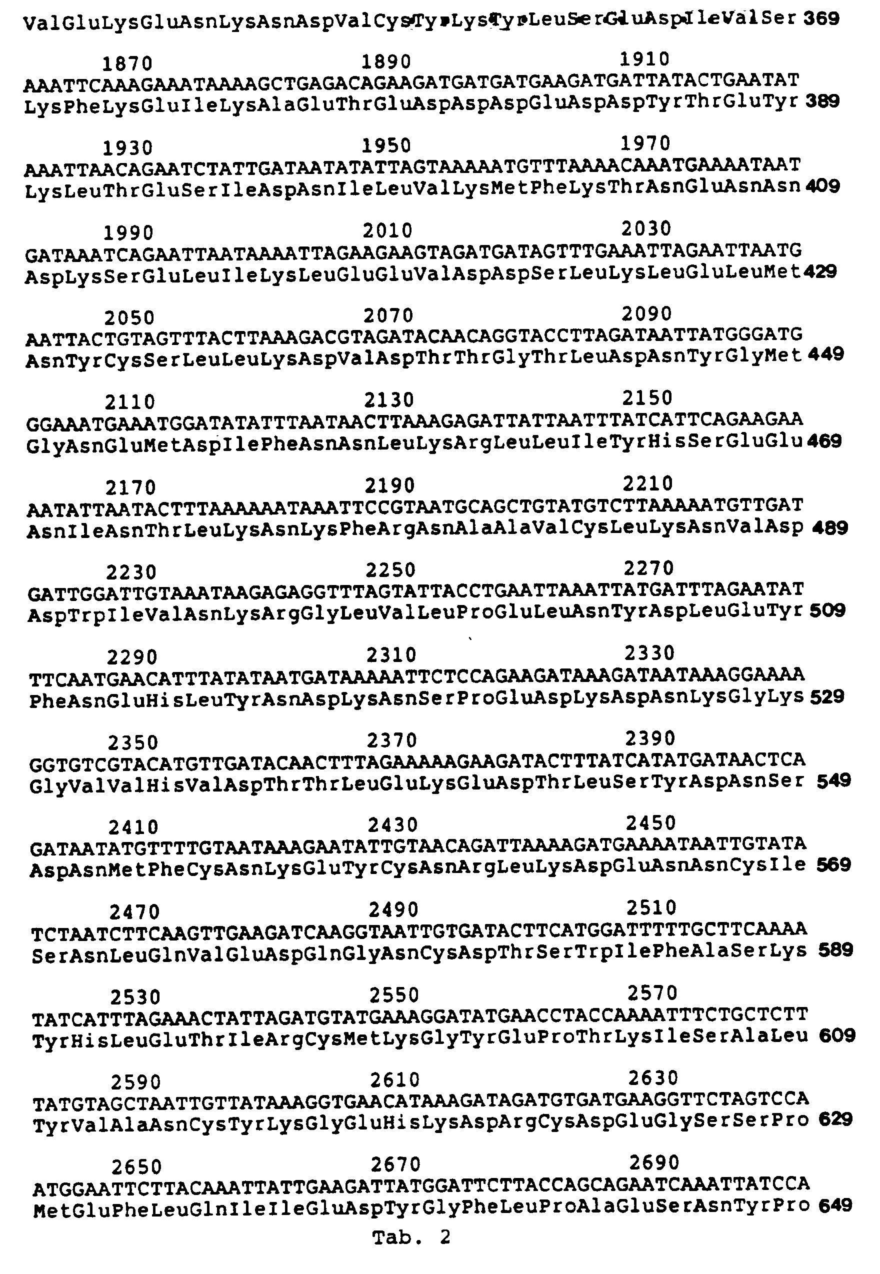

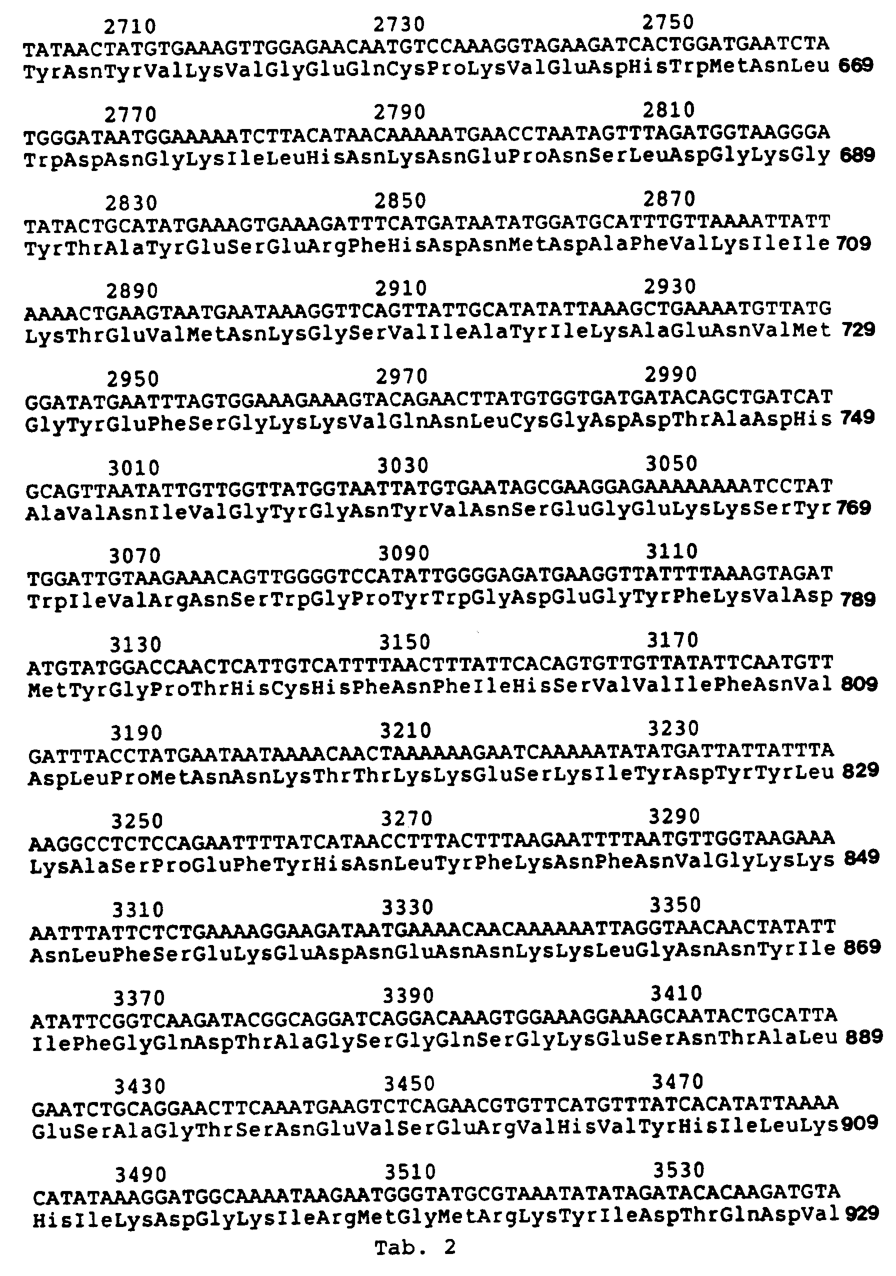

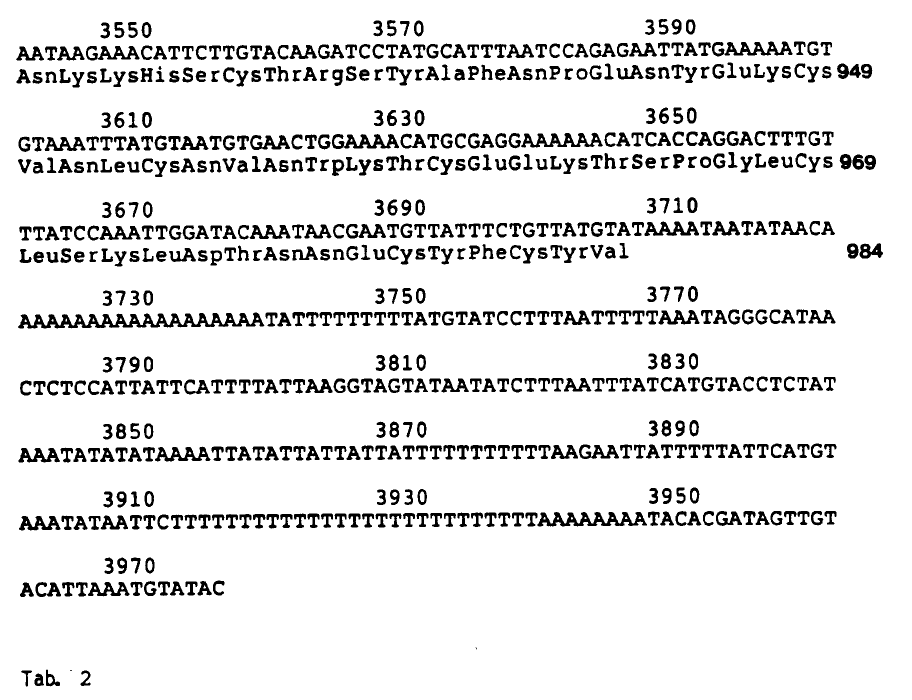

Komplette Nukleotidsequenz des 140 kD Gens und abgeleitete Aminosäuresequenz des 140 kD Antigens des P.falciparum Stammes FCBR. Die Nukleotide sind im Abstand von 20 Basen oberhalb des codogenen DNA-Stranges numeriert. Die Aminosäurepositionen des Antigens sind rechts angegeben. Die Intronsequenzen sind durch Pfeile markiert. Repetitive Sequenzabschnitte sind unterstrichen.

## Patentansprüche (Patentansprüche für folgende(n) Vertragsstaat(en): AT BE CH DE FR GB GR IT LI LU NL und SE)

1. Fragmente des 140 kd Antigens von *Plasmodium falciparum*-Merozoiten und -Schizonten mit den in Tabelle 2 angegebenen Aminosäureteilsequenzen der Positionen 1882 bis 1917, 2314 bis 2403 und 2602 bis 2631, die hydrophile Oberflächenbereiche sind, die potentielle antigene Epitope darstellen.

2. Isolierte DNA- oder RNA-Sequenz, kodierend für mindestens ein Fragment nach Anspruch 1.

3. Vektor, enthaltend mindestens eine Nukleotidsequenz nach Anspruch 2.

4. Wirtszelle, enthaltend einen Vektor nach Anspruch 3.

5. Diagnostikum, enthaltend mindestens eine Nukleotidsequenz nach Anspruch 2.

6. Verfahren zur gentechnischen Herstellung von mindestens einem Teil des 140 kd Proteins, der ein hydrophiler Oberflächenbereich ist, der einen potentiellen antigenen Epitop darstellt, dadurch gekennzeichnet, daß mindestens eine Nukleotidsequenz nach Anspruch 2 exprimiert und das Expressionsprodukt isoliert wird.

## Patentansprüche (Patentansprüche für folgende(n) Vertragsstaat(en): ES)

1. Verfahren zur Herstellung von Fragmenten des 140 kd Antigens von *Plasmodium falciparum*-Merozoiten und -Schizonten mit den in Tabelle 2 angegebenen Aminosäureteilsequenzen der Positionen 1882 bis 1917, 2314 bis 2403 und 2602 bis 2631, die hydrophile Oberflächenbereiche sind, die potentielle antigene Epitope darstellen.

2. Verfahren zur Herstellung einer isolierten DNA- oder RNA-Sequenz, kodierend für mindestens ein Fragment nach Anspruch 1.

3. Verfahren zur Herstellung eines Vektors, enthaltend mindestens eine Nukleotidsequenz nach Anspruch 2.

4. Verfahren zur Herstellung einer Wirtszelle, enthaltend einen Vektor nach Anspruch 3.

5. Verfahren zur Herstellung eines Diagnostikums, enthaltend mindestens eine Nukleotidsequenz nach Anspruch 2.

6. Verfahren zur gentechnischen Herstellung von mindestens einem Teil des 140 kd Proteins, der ein hydrophiler Oberflächenbereich ist, der einen potentiellen antigenen Epitop darstellt, dadurch gekennzeichnet, daß mindestens eine Nukleotidsequenz nach Anspruch 2 exprimiert und das Expressionsprodukt isoliert wird.

## Claims (Claims for the following Contracting State(s): AT BE CH DE FR GB GR IT LI LU NL and SE)

1. Fragments of the 140 kd antigen of *Plasmodium falciparum* merozoites and schizonts having the partial amino acid sequences of positions 1882 to 1917, 2314 to 2403 and 2602 to 2631 which are indicated in Table 2 and which are hydrophilic surface regions representing potential antigenic epitopes.

2. Isolated DNA or RNA sequence coding for at least one fragment according to Claim 1.

3. Vector containing at least one nucleotide sequence according to Claim 2.

4. Host cell containing a vector according to Claim 3.

5. Diagnostic aid containing at least one nucleotide sequence according to Claim 2.

6. Process for the preparation by genetic manipulation of at least one part of the 140 kd proteins, which part is a hydrophilic surface region representing a potential antigenic epitope, characterized in that at least one nucleotide sequence according to Claim 2 is expressed, and the expression product is isolated.

## Claims (Claims for the following Contracting State(s): ES)

1. Process for preparing fragments of the 140 kd antigen of *Plasmodium falciparum* merozoites and schizonts having the partial amino acid sequences of positions 1882 to 1917, 2314 to 2403 and 2602 to 2631 which are indicated in Table 2 and which are hydrophilic surface regions representing potential antigenic epitopes.

2. Process for preparing an isolated DNA or RNA sequence coding for at least one fragment according to Claim 1.

3. Process for preparing a vector containing at least one nucleotide sequence according to Claim 2.

4. Process for preparing a host cell containing a vector according to Claim 3.

5. Process for preparing a diagnostic aid containing at least one nucleotide sequence according to Claim 2.

6. Process for the preparation by genetic manipulation of at least one part of the 140 kd proteins, which part is a hydrophilic surface region representing a potential antigenic epitope, characterized in that at least one nucleotide sequence according to Claim 2 is expressed, and the expression product is isolated.

## Revendications (Revendications pour l'(les) Etat(s) contractant(s) suivant(s): AT BE CH DE FR GB GR IT LI LU NL et SE)

1. Fragments de l'antigène de 140 kDa de mérozoïtes et schizontes de *Plasmodium falciparum*, ayant les séquences partielles d'aminoacides des positions 1882 à 1917, 2314 à 2403 et 2602 à 2631 indiquées dans le tableau 2, qui sont des régions superficielles hydrophiles qui représentent des épitopes antigéniques potentiels.

2. Séquence d'ADN ou d'ARN isolée, codant pour au moins un fragment selon la revendication 1.

3. Vecteur, contenant au moins une séquence nucléotidique selon la revendication 2.

4. Cellule hôte, contenant un vecteur selon la revendication 3.

5. Agent de diagnostic, contenant au moins une séquence nucléotidique selon la revendication 2.

6. Procédé pour la production par génie génétique d'au moins une partie de la protéine de 140 kDa, qui est une région superficielle hydrophile qui représente un épitope antigénique potentiel, caractérisé en ce qu'au moins une séquence nucléotidique selon la revendication 2 est exprimée et le produit d'expression est isolé.

## Revendications (Revendications pour l'(les) Etat(s) contractant(s) suivant(s): ES)

1. Procédé pour la production de fragments de l'antigène de 140 kDa de mérozoïtes et schizontes de *Plasmodium falciparum*, ayant les séquences partielles d'aminoacides des positions 1882 à 1917, 2314 à 2403 et 2602 à 2631 indiquées dans le tableau 2, qui sont des régions superficielles hydrophiles qui représentent des épitopes antigéniques potentiels.

2. Procédé pour la production d'une séquence d'ADN ou d'ARN isolée, codant pour au moins un fragment selon la revendication 1.

3. Procédé pour la production d'un vecteur contenant au moins une séquence nucléotidique selon la revendication 2.

4. Procédé pour la production d'une cellule hôte contenant un vecteur selon la revendication 3.

5. Procédé pour la production d'un agent de diagnostic contenant au moins une séquence nucléotidique selon la revendication 2.

6. Procédé pour la production par génie génétique d'au moins une partie de la protéine de 140 kDa, qui est une région superficielle hydrophile qui représente un épitope antigénique potentiel, caractérisé en ce qu'au moins une séquence nucléotidique selon la revendication 2 est exprimée et le produit d'expression est isolé.
